# EUROPÄISCHE PATENTSCHRIFT

(11) **EP 1 301 501 B1**
(45) Veröffentlichungstag und Bekanntmachung des Hinweises auf die Patenterteilung: **31.03.2004**
(21) Anmeldenummer: 01969391.0
(22) Anmeldetag: 13.07.2001
(51) Int. Cl.: C07D 307/04, A61K 31/34, A61P 3/10

(54) **TOCOTRIENOLCHINON- ZYKLISIERUNGSPRODUKTE MIT ANTIHYPERCHOLESTERIN WIRKUNG**
TOCOTRIENOLQUINONE CYCLISATION PRODUCT WITH AN ANTI-HYPERCHOLESTEROL EFFECT
PRODUITS DE CYCLISATION DE LA TOCOTRIENOLQUINONE A EFFET ANTIHYPERCHOLESTEROL

(30) Priorität: 14.07.2000 DE 10034233
(43) Veröffentlichungstag der Anmeldung: 16.04.2003
(73) Patentinhaber: BASF AKTIENGESELLSCHAFT, 67056 Ludwigshafen (DE)
(72) Erfinder: BALDENIUS, Kai-Uwe, 67063 Ludwigshafen (DE); SCHRÖDER, Hartwig, 59226 Nussloch (DE); KRÄMER, Klaus, 76829 Landau (DE); SCHEIN, Karin, 67065 Kudwigshafen (DE); STÜRMER, Rainer, 67127 Rödersheim-Gronau (DE)
(74) Vertreter: Pohl, Michael, Dr.
(86) Internationale Anmeldenummer: PCT/EP2001/008163
(87) Internationale Veröffentlichungsnummer: WO 2002/006261

(56) Entgegenhaltungen:
- EP-A- 0 421 419
- EP-A- 0 476 493
- EP-A- 0 571 928
- EP-A- 0 669 132
- EP-A- 0 882 450
- US-A- 5 318 993
- KOMIYAMA K ET AL: "STUDIES ON THE BIOLOGICAL ACTIVITY OF TOCOTRIENOLS" STN CHEMICAL ABSTRAC, XP002914546

## Beschreibung

Die Akkumulation von Cholesterin im Kreislauf bzw. der hohe Cholesterin- und LDL-Cholesterinspiegel im Plasma als Folge einer Nahrung, die einen hohen Gehalt an gesättigten Fettsäuren und Cholesterin aufweist, korreliert mit der Inzidenz der koronaren Herzkrankheit. Diese ist eine der häufigsten Todesursachen in den Industrienationen. Hypercholesterinämie spielt auch eine kausale Rolle bei der Pathogenese von Arteriosklerose, Leberkrebs, Xanthomatose und vieler anderer Krankheiten.

Wirksame, in der Medizin eingesetzte Hemmstoffe der Cholesterinbiosynthese sind die Statine, wie Atorvastatin, Fluvastatin, Cerivastatin, Lovastatin, Mevastatin, Pravastatin und Simvastatin. Darüber hinaus zeigen die im Knoblauchextrakt enthaltenen Wirkstoffe Allicin und Ajoene, das im Artischokenextrakt enthaltene Luteolin sowie auch Fibrate und das Piperizin-Derivat BM 15.766 eine Cholesterin-senkende Wirkung (Gebhardt, R. et al. (1994) Biochim. Biophys. Acta 1213: 57-63; Gebhardt, R. (1998) J. Pharmacol. Exp. Therap. 286: 1122-1128; Aufenanger, J. et al. (1985) Biochem. Pharmacol. 35(6): 911-916). Auch Palmöl zeichnet sich durch eine Cholesterin-senkende Wirkung aus, trotz seines hohen Gehalts an gesättigten Fettsäuren. In Palmöl sind Tocopherole (wie z. B. das Vitamin E) und Tocotrienole enthalten, die für diese Wirkung verantwortlich gemacht wurden (US 5,217,992). Tocopherole und Tocotrienole unterscheiden sich in erster Linie dadurch, dass Tocopherole eine gesättigte, Tocotrienole dagegen eine ungesättigte Seitenkette besitzen, die jeweils für die Bindung an Lipoproteine und an Membrane verantwortlich ist (Hood, R. L. (1998) Phytochemicals, 33-41). Tocopherole werden aufgrund ihrer antioxidativen Wirkung als Mittel zur Prophylaxe vor oxidativen Schäden des Muskels, der Haut, der Haare, des Immunsystems und bei Rauchern eingesetzt. Außerdem verwendet man sie als Anticancerogene, als Nitritfänger, z. B. in Räucherschinken, und in der Veterinärmedizin (Römpp-Lexikon Chemie (1999), Georg Thieme Verlag, Stuttgart, 4572-4573). Sie üben jedoch keinen Einfluss auf den Lipidmetabolismus aus, obwohl lange Zeit Vitamin E die Wirkung zugeschrieben wurde, die Oxidation von LDL-Cholesterin zu verhindern. Dagegen vermögen die im Palmöl enthaltenen Tocotrienole den Plasmacholesterinspiegel zu senken. Die hemmende Wirkung z. B. des γ-Tocotrienols auf die Cholesterinsynthese kommt durch eine direkte Farnesylierung der HMGR oder durch eine Feedback-Hemmung der HMGR durch ein farnesyliertes Protein zustande (Guthrie, N. and K. K. Caroll (1998), Biol. Oxidants and Antioxidants: Molecular Mechanisms and Health Effects, Champaign, AOCS-Press, 257-264; Qureshi, N. (1993) Vitamin E in Health and Disease, 247-267). Eine Zusammensetzung aus γ-Tocotrienol und Lovastatin, das über einen anderen, unabhängigen Mechanismus wirkt und dessen Wirkung dadurch additiv ist, ist noch effektiver. Zur Bestimmung des Einflusses eines Wirkstoffs auf die Cholesterinsyntheserate wird ein in vitro-Test herangezogen, bei dem es bei Hemmung der HMGR, z. B. durch γ-Tocotrienol, in der Mikrosomenfraktion aus HepG2-Zellen zu einem verminderten ¹⁴C-Acetateinbau kommt.

Palmöl und die darin enthaltenen Tocotrienole besitzen also in Vögeln und Säugern eine vorteilhafte, senkende Wirkung auf den Cholesterin- und LDL-Cholesterinspiegel im Plasma, ohne den HDL-Spiegel zu beeinflussen. Darüber hinaus senken Tocotrienole den Apolipoprotein-B-Spiegel, ebenfalls ein Maß für den Cholesterinspiegel im Blut (Brown et al. (1980), J. Lipid Res. 21:505-517). Die US 5,318,993 beschreibt eine Stoffgruppe von synthetischen Tocotrienol-Analogen, ringgeöffnete Chinone, die nach Oxidation von Tocotrienolen entstehen und sich in vitro (¹⁴C-Acetateinbau in die Mikrosomenfraktion aus HepG2-Zellen) und in vivo (Cholesterin- und LDL-Cholesterinspiegel in Hühnern) durch eine erhöhte Wirksamkeit im Vergleich zu Tocotrienolen auszeichnen.

Die prophylaktische Wirkung der Tocotrienole bei der Ateriosklerose beruht neben der Cholesterin-senkenden Wirkung auf der Hemmung der Plättchenaggregation. So sinken bei Tocotrienolgabe die Plasmaspiegel von Thromboxan B2 und Plättchenfaktor IV (Wright et al., A Symposium on Drugs Affecting Lipid Metabolism, Houston, Texas (Nov. 1989); Papas, A. M. (1999), CRC, Boca Raton, Florida, S. 189-210).

Den Tocotrienolen wird darüber hinaus eine anticancerogene Wirkung zugeschrieben. So vermag Palmöl die Entstehung von Leberkarzinomen zu verhindern, während ein Mangel von Tocotrienolen im Fettgewebe die Inzidenz von Brust- und Hautkrebs erhöht. Die anticancerogene Wirkung der Tocotrienole bei Leberkrebs beruht ebenfalls auf der Hemmung der Cholesterinsynthese und damit auf einer wachstums- und proliferationshemmenden Wirkung, die z. B. bei A549-Zellen nachgewiesen wurde (Hood, R. L. (1998), Phytochemicals, 33-51; Watkins, T. R. et al. (1999), CRC, Boca Raton, Florida, 479-496; Bennis F. et al. (1993), Int. J. Cancer 55:640-645). Eine bedeutendere Rolle für die anticancerogene Wirkung der Tocotrienole spielt jedoch deren 6'-OH-Gruppe, die aufgrund ihrer antioxidativen Eigenschaften als Radikalfänger wirkt. Außerdem hemmen Tocotrienole die Proteinkinase C, die z. B. bei der Tumorentwicklung, Zellproliferation und -differenzierung, Plättchenaggregation und Radikalfreisetzung involviert sein kann. Schließlich wird vermutlich über eine Farnesylierung von ras dessen Cancerogenität verhindert (Hood, R. L. (1998), Phytochemicals, 33-51; Watkins, T. R. et al. (1999), CRC, Boca Raton, Florida, 479-496). Aufgrund dieser anticancerogenen Eigenschaften können Tocotrienole in Kombination mit Tamoxifen und Hesperatin (Flavonoide) wirksam zur Tumorprophylaxe und -therapie eingesetzt werden (Hood, R. L. (1998), Phytochemicals, 33-51; Guthrie, N. and K. K. Caroll (1998), Biol. Oxidants and Antioxidants, Molecular Mechanism and Health Effects, Champaign, AOCS-Press, 257-264).

Obwohl ringgeöffnete Chinone eine gegenüber den natürlich vorkommenden Tocotrienolen verbesserte Wirkung zeigen (US 5,318,993), stellte sich das Problem, noch wirksamere synthetische Analoge bereitzustellen.

Diese Aufgabe wurde überraschenderweise durch Bereitstellung synthetischer Tocotrienolchinon-Cyclisierungsprodukte, im weiteren Verlauf als Cyclisierungsprodukte bezeichnet, mit einer erhöhten Wirksamkeit im Vergleich zu γ-Tocotrienol oder den ringgeöffneten Chinonen gelöst. Besonders überraschend war vor allen Dingen die Tatsache, dass die erfindungsgemäßen Cyclisierungsprodukte bereits in geringen Konzentrationen von 10⁻⁶ bis 10⁻⁵ M (Mol/l) eine signifikant hohe Wirksamkeit entfalten.

Die Erfindung betrifft insbesondere Verbindungen der Formel I 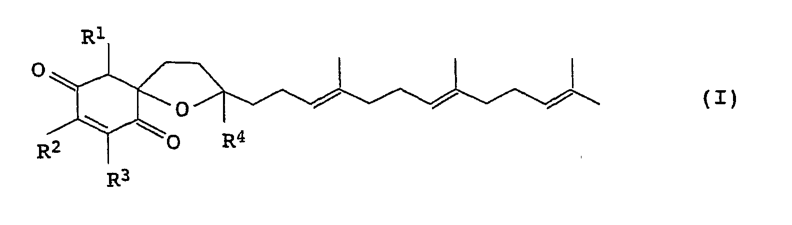 wobei
R¹, R², R³ und R⁴ voneinander unabhängig für H oder C₁-C₆-Alkyl stehen;
R¹ bevorzugt für H oder CH₃, besonders bevorzugt für H steht;
R² bevorzugt für H oder CH₃, besonders bevorzugt für CH₃ steht;
R³ bevorzugt für CH₃ und
R⁴ bevorzugt für H oder CH₃ steht; in stereoisomerenreiner Form oder als Gemisch von Stereoisomeren. Außerdem kann R¹ für Halogene, insbesondere Cl stehen.

C₁-C₆-Alkyl umfasst Methyl, Ethyl, n- und i-Propyl, n-, i- und t-Butyl sowie n-Pentyl und n-Hexyl und deren verzweigte Analoge.

Die Erfindung umfasst insbesondere auch die beiden Diastereomeren (polar/unpolar) sowie die Enantiomere der Verbindungen der Formel I. Das polare Diastereomer zeichnet sich dadurch aus, dass es von einer Kieselgelsäule durch ein hauptsächlich aus einem Alkan bestehenden Lösungsmittelgemisch langsamer eluiert wird als das unpolare Diastereomer.

Gegenstand der Erfindung ist außerdem ein Verfahren zur Herstellung einer Verbindung der Formel I, dadurch gekennzeichnet, dass man ein Tocotrienolchinon der Formel III, 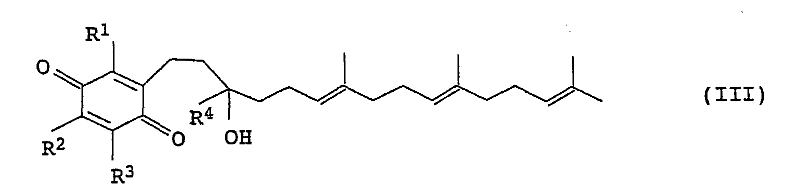 worin R¹ bis R⁴ die in Formel I angegebenen Bedeutungen besitzen, in Gegenwart eines Katalysators zu einer Verbindung der Formel I umsetzt. Dabei erhält man das zum eingesetzten Tocotrienolchinon korrespondierende Tocotrienolchinon-Cyclisierungsprodukt, d. h. die Reste R¹ bis R⁴ werden durch das eingesetzte Tocotrienolchinon vorgegeben.

Bevorzugte Katalysatoren sind Alkalicarbonate, beispielsweise Natrium- und Kaliumcarbonat, besonders bevorzugt ist Cäsiumcarbonat. Dazu werden 10 bis 200 g/l, vorzugsweise 100 g/l, eines Tocotrienolchinons der Formel III und 5 bis 15 g/l, vorzugsweise 10 g/l eines der oben genannten Katalysatoren in einem organischen Lösungsmittel, vorzugsweise Methylenchlorid, gelöst und umgesetzt.

Die Umsetzung kann in gängigen Reaktionsgefäßen, vorzugsweise Glasreaktoren, erfolgen, unter Verwendung üblicher Rühr- und Mischvorrichtungen. Die Reaktionsdauer sollte so gewählt werden, dass es zu einer vollständigen Umsetzung des Tocotrienolchinons zum korrespondierenden Cyclisierungsprodukt kommt, bevorzugt 1 bis 6 Stunden. Die Reaktion erfolgt bei etwa 10 bis 100 °C, vorzugsweise bei 20 bis 50 °C. Anschließend lässt sich beispielsweise durch Zugabe von n-Heptan (im Verhältnis 2:1, bezogen auf das Volumen der Ausgangslösung) und einer 10 %igen Natriumhydrogensulfatlösung (im Verhältnis 1:1, bezogen auf das Volumen der Ausgangslösung) eine Phasentrennung erreichen, wobei das Cyclisierungsprodukt und bei der Umsetzung entstehende Nebenkomponenten in der organischen Phase zu finden sind.

Die Reaktionslösung bzw. die vorstehende organische Phase können zur weiteren Reinigung des Cyclisierungsprodukts, z. B. nach Trocknung über Natriumsulfat eingedampft und z. B. chromatographisch weiter gereinigt werden.

Beispielsweise kann das gewünschte Produkt mit Hilfe einer Kieselgelsäule von Nebenkomponenten getrennt werden. Dabei wird, wie oben erwähnt, das polare Diastereomer durch ein hauptsächlich aus einem Alkan bestehenden Lösungsmittel langsamer von der Säule eluiert als das unpolare Diastereomer. Man erreicht durch dieses oder entsprechende chromatographische Verfahren eine teilweise oder vollständige Trennung der beiden Diastereomere. Dies ist jedoch nicht immer notwendig, da beide Diastereomere eine hohe Cholesterin-senkende Wirkung aufweisen.

Das im erfindungsgemäßen Verfahren eingesetzte Tocotrienolchinon der Formel III kann in einem, dem beschriebenen erfindungsgemäßen verfahren vorausgehenden Syntheseverfahren, einer Oxidationsreaktion, aus dem korrespondieren Tocotrienol der Formel II 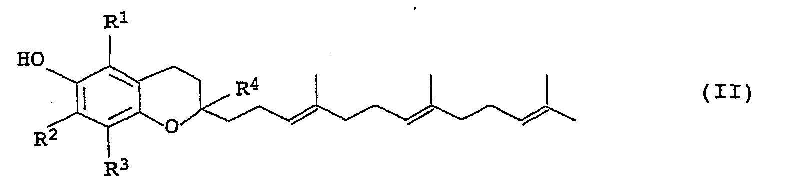 worin R¹ bis R⁴ die in Formel I angegebenen Bedeutungen besitzen, hergestellt werden. Die Reste R¹ bis R⁴ des resultierenden Tocotrienolchinons werden durch das eingesetzte Tocotrienol vorgegeben. Tocotrienole können synthetisch hergestellt oder aus natürlichen Quellen isoliert werden (Khor H.T. and J.Y. Lai (1991), Biologic Oxidants and Antioxidants, AOCS Press, 267-273; Goh S.H. et al. (1991) Biologic oxidants and Antioxidants, AOCS Press, 274-283; Qureshi N. (1993), Vitamin E in Health and Disease, 247-267). Die Umsetzung des Tocotrienols zu Tocotrienolchinon erfolgt durch Zugabe einer wässrigen Lösung eines Oxidationsmittels, ausgewählt unter Cer(IV)-Salzen oder Fe(III)-Salzen, vorzugsweise Ammoniumcer(IV)nitrat oder Ammoniumeisen(III)nitrat in einer Konzentration von beispielsweise 2 bis 5 Mol Oxidationsmittel/Mol Tocotrienol, bevorzugt 2 bis 3 Mol/Mol. Die Umsetzung kann in allen gängigen Reaktionsgefäßen bei Raumtemperatur bei einer Reaktionsdauer von 0,5 bis 8 Stunden, vorzugsweise 1 Stunde, erfolgen.

Die oben beschriebenen erfindungsgemäßen Reaktionen werden außerdem bevorzugt in einer Inertgasatmosphäre, wie z. B. unter Stickstoff, durchgeführt.

Gegenstand der Erfindung ist auch die Verwendung von Verbindungen der Formel I als Nahrungsergänzungsmittel. So können erfindungsgemäße Cyclisierungsprodukte in Speisen und Getränken enthalten sein bzw. vor, während oder nach der Mahlzeit beispielsweise in Kombinationspräparaten mit anderen Nahrungsergänzungsmitteln eingenommen werden.

Die vorliegende Erfindung umfasst weiterhin Verbindungen der Formel I zur medizinischen Anwendung.

Erfindungsgemäße Cyclisierungsprodukte finden Verwendung bei der Herstellung von Medikamenten und Nahrungsergänzungsmitteln zur Senkung des Plasmacholesterinspiegels bei Vögeln und bei Säugetieren, insbesondere beim Menschen. So werden erfindungsgemäße Medikamente in der Human- und Veterinärmedizin angewendet, und auf Grundlage der erfindungsgemäßen Cyclisierungsprodukte lassen sich Medikamente herstellen, die zur Behandlung von Krankheiten im Zusammenhang mit Hypercholesterinämie verwendet werden können. Ferner können erfindungsgemäße Medikamente auch zur Vorbeugung solcher Krankheiten eingesetzt werden.

Grundlage der Wirksamkeit ist möglicherweise die hemmende Wirkung der erfindungsgemäßen Cyclisierungsprodukte auf die 3-Hydroxy-3-methylglutaryl-CoA-Reduktase (HMGR), das Geschwindigkeits-bestimmende Enzym der Cholesterinbiosynthese.

Weiter finden die Cyclisierungsprodukte Verwendung bei der Herstellung von Medikamenten und Nahrungsergänzungsmitteln zur Behandlung und zur Vorbeugung von Krebserkrankungen.

Gegenstand der Erfindung sind auch pharmazeutische Mittel, umfassend eine pharmazeutisch wirksame Menge wenigstens einer erfindungsgemäßen Verbindung der Formel I und wenigstens einen pharmazeutisch verträglichen Träger. Dieser Träger kann abhängig von der erwünschten Dosierungsform in fester oder flüssiger Form vorliegen. Bevorzugte Träger sind Stärke, Lactose, Sucrose und Speiseöle.

Erfindungsgemäße Medikamente und Nahrungsergänzungsmittel umfassen auch Zusammensetzungen oder Kombinationspräparate der Cyclisierungsprodukte mit anderen Wirkstoffen. Vorteilhafterweise können dadurch die Dosierungen der einzelnen Wirkstoffe herabgesetzt werden.

Bevorzugt für die Verwendung der Cyclisierungsprodukte zur Herstellung eines Medikaments zur Behandlung und Vorbeugung von Krankheiten im Zusammenhang mit Hypercholesterinämie sind Zusammensetzungen oder Kombinationspräparate. Derartige Kombinationspräparate können weitere Inhibitoren der HMGR enthalten, deren hemmender Wirkung ein vergleichbarer oder anderer Mechanismus zugrunde liegt und aus diesem Grund eine erhöhte Wirksamkeit zu erwarten ist. Demgemäß können erfindungsgemäße Zusammensetzungen beispielsweise Atorvastatin, Fluvastatin, Cerivastatin, Lovastatin, Mevastatin, Pravastatin, Simvastatin, SRI-62320, Probucol, Niacin (Nikotinsäure), Allicin, Ajoene, Luteolin, BM 15.766, Isoflavone, Curcumin, Phytosterole, n-3 PUFA, Probiotika und Cholesterin-senkende Fibrate und deren Derivate und Konjugate einzeln oder in Kombination als weitere Wirkstoffe enthalten. Bevorzugt für die Verwendung der erfindungsgemäßen Cyclisierungsprodukte zur Herstellung eines Medikaments zur Behandlung und Vorbeugung von Krebserkrankungen sind Zusammensetzungen oder Kombinationspräparate, die die Wirkstoffe Tamoxifen und Flavonoide, wie Hesperatin oder dergleichen enthalten.

Erfindungsgemäße Medikamente, Zusammensetzungen und Kombinationspräparate können in Form von Tabletten, Granulaten, Pulver, Dragees, Pastillen, Pellets, Kapseln, Zäpfchen, Gelen, Lösungen, Emulsionen und Suspensionen zur enteralen und parenteralen Verabreichung vorliegen. Außerdem können die erfindungsgemäßen Verbindungen in Gelen, Lotionen und Cremes zur kutanen Applikation enthalten sein. Die Cyclisierungsprodukte können auch als Nahrungsergänzungsmittel in Speisen und Getränken enthalten sein bzw. als Kombinationspräparate mit anderen Nahrungsergänzungsmitteln in oben genannter Form vorliegen. Gamma-Tocotrienol-Cyclisierungsprodukt kann in einer Dosierung von 0,5 bis 2000 mg/d, jedoch auch in höheren oder niedrigeren Dosierungen, verabreicht werden. Die genaue Dosierung und das Behandlungsmuster wird vom Zustand des Patienten, der Schwere und dem Verlauf der Krankheit, sowie der Disposition des Patienten für diese Krankheit abhängen und wird geeigneterweise vom behandelnden Arzt genau festgelegt. Entsprechendes gilt für die Verwendung in der Veterinärmedizin.

Die Erfindung wird nun in den folgenden nichtlimitierenden Beispielen und unter Bezugnahme auf beiliegende Figuren näher beschrieben. Dabei zeigt
- Fig. 1: den ¹⁴C-Acetat-Einbau in HepG2-Zellen bei steigenden Konzentrationen von R-γ-Tocotrienol
- Fig. 2: den ¹⁴C-Acetat-Einbau in HepG2-Zellen bei steigenden Konzentrationen von γ-Tocotrienolchinon
- Fig. 3: den ¹⁴C-Acetat-Einbau in HepG2-Zellen bei steigenden Konzentrationen von α-Tocotrienolchinon
- Fig. 4: den ¹⁴C-Acetat-Einbau in HepG2-Zellen bei steigenden Konzentrationen des polaren Diastereomers des Cyclisierungsprodukts, worin R¹ für H und R², R³ und R⁴ jeweils für CH₃ steht (γ-Tocotrienolchinon-Cyclisierungsprodukt)
- Fig. 5: den ¹⁴C-Acetat-Einbau in HepG2-Zellen bei steigenden Konzentrationen des unpolaren Diastereomers des Cyclisierungsprodukts, worin R¹ für H und R², R³ und R⁴ jeweils für CH₃ steht (γ-Tocotrienolchinon-Cyclisierungsprodukt)
- Fig. 6: den ¹⁴C-Acetat-Einbau in HepG2-Zellen bei steigenden Konzentrationen des Diastereomerengemischs des Cyclisierungsprodukts, worin R¹ für H und R², R³ und R⁴ jeweils für CH₃ steht (γ-Tocotrienolchinon-Cyclisierungsprodukt)

Das folgende Beispiel erläutert die Synthese der erfindungsgemäßen Verbindung, ohne sie zu beschränken.

Beispiel 1: Synthese von γ-Tocotrienolchinon-Cyclisierungsprodukt (Verbindung der Formel I, worin R¹ = H, R² = CH₃, R³ = CH₃ und R⁴ = CH₃)

In einem 6 l-Glaskolben mit Scheibenrührer, Kühler, Tropftrichter und Thermometer wurde unter N₂-Schutz R-γ-Tocotrienol (gamma-T3, 100,0 g/0,24 Mol) in Ethanol z. A. (4800 ml) gelöst. Zu dieser Lösung wurde bei Raumtemperatur Ammoniumcer(IV)nitrat (307,2 g/0,56 Mol), gelöst in destilliertem Wasser (400 ml), zugegeben. Dieses Gemisch wurde eine Stunde bei Raumtemperatur gerührt und eine DC-Probe entnommen. Diese zeigte vollständigen Umsatz des R-γ-Tocotrienols.

Danach wurde der Ansatz zwischen n-Heptan (800 ml) und destilliertem Wasser (8000 ml) verteilt. Die Phasen wurden getrennt und die wässrige Phase noch einmal mit n-Heptan (800 ml) extrahiert.

Die organischen Phasen wurden vereint und zweimal mit destilliertem Wasser (2000 ml) gewaschen. Zur besseren Phasentrennung wurde bei der zweiten Wäsche Essigsäure z. A. (ca. 3 ml/ca. 0,05 Mol) zugegeben. Anschließend wurde die organische Phase über Natriumsulfat getrocknet. Durch vollständiges Einengen an einem Rotationsverdampfer wurde γ-Tocotrienolchinon (104,5 g) erhalten. Dieses wurde wie folgt weiter umgesetzt.

In einem 4 1-Glasreaktor mit Kühler, Scheibenrührer und Thermometer wurden unter N₂-Schutz γ-Tocotrienolchinon (104,5 g), Cäsiumcarbonat (Cs₂CO₃, 10,0 g/0,03 Mol) und Methylenchlorid (CH₂Cl₂, 1000 ml) zusammengegeben und bei Rückfluss gerührt. Nach 5 Stunden wurde eine DC-Probe entnommen. Diese zeigte weitgehenden Umsatz, aber auch Nebenkomponenten.

Es wurden n-Heptan (2000 ml) und 10 %ige Natriumhydrogensulfat-Lösung (1000 ml/0,83 Mol) zugegeben und kurz bei höherer Drehzahl gerührt. Anschließend wurden die Phasen getrennt und die organische Phase noch zweimal mit destilliertem Wasser (1000 ml) gewaschen. Danach wurde die organische Phase über Natriumsulfat getrocknet und durch vollständiges Einengen an einem Rotationsverdampfer wurde γ-Tocotrienolchinon-Cyclisierungsprodukt (100,7 g) erhalten.

### Trennung der Diastereomere:

Dieses wurde vollständig über eine Flash 150-Kieselgelsäule der Firma Biotage chromatographiert. Als Laufmittel wurden 6 % Ethylacetat in n-Heptan benutzt. Nach vollständigem Einengen der Hauptfraktionen (DC-Kontrolle) am Rotationsverdampfer wurde γ-Tocotrienolchinon-Cyclisierungsprodukt (0,12 Mol; 49,4 g) erhalten. Ein DC vom Endprodukt zeigte nur die beiden Diastereomere und keine Nebenprodukte mehr. Trennung der Diastereomere: Zwei Nebenfraktionen der Chromatographie, die jeweils nur eines der beiden Diastereomeren enthielten, wurden ebenfalls mittels Rotationsverdampfer vollständig eingeengt. Dadurch wurden unpolares Diastereomer (0,82 g; RF-Wert: 0,39 (Laufmittel: 30 % MTB/n-Heptan); Drehwert[α]²⁵_{D}:+2,0° (c=1, Ethanol)) und polares Diastereomer (0,86 g; RF-Wert: 0,35(Laufmittel: 30 % MTB/n-Heptan); Drehwert [α]²⁵_{D}:-40,0° (c=1, Ethanol)) erhalten.
¹³C-NMR-Daten der beiden Diastereomere (in CDCl₃/DMSO-d₆ 1:4): Chemische Verschiebung in δ ppm (TMS), Multiplizität (S = Singulett, D = Dublett, T = Triplett, Q = Quartett)

### Diastereomer 1:

| | | | | | | | | | |
|---|---|---|---|---|---|---|---|---|---|
| 1. | 85,14 S | 7. | 144,24 S | 13. | 134,08 S | 19. | 26,14 T | 25. | 17,50 Q |
| 2. | 35,83 T | 8. | 144,82 S | 14. | 39,37 T | 20. | 124,17 D | 26. | 25,97 Q |
| 3. | 32,82 T | 9. | 196,68 S | 15. | 26,35 T | 21. | 130,46 S | 27. | 12,53 Q |
| 4. | 85,25 S | 10. | 40,33 T | 16. | 123,94 D | 22. | 25,51 Q | 28. | 13,12 Q |
| 5. | 50,43 T | 11. | 22,92 T | 17. | 134,23 S | 23. | 15,69 Q | | |
| 6. | 195,13 S | 12. | 124,17 D | 18. | 39,37 T | 24. | 15,76 Q | | |

### Diastereomer 2:

| | | | | | | | | | |
|---|---|---|---|---|---|---|---|---|---|
| 1. | 85,25 S | 7. | 144,24 S | 13. | 134,26 S | 19. | 26,14 T | 25. | 17,50 Q |
| 2. | 36,11 T | 8. | 144,62 S | 14. | 39,37 T | 20. | 124,17 D | 26. | 26,68 Q |
| 3. | 32,53 T | 9. | 196,39 S | 15. | 26,35 T | 21. | 130,45 S | 27. | 12,46 Q |
| 4. | 85,30 S | 10. | 40,85 T | 16. | 123,94 D | 22. | 25,51 Q | 28. | 13,08 Q |
| 5. | 50,69 T | 11. | 22,83 T | 17. | 134,23 S | 23. | 15,63 Q | | |
| 6. | 195,16 S | 12. | 124,28 D | 18. | 39,37 T | 24. | 15,78 Q | | |

### Gaschromatographische Analyse:

Die GC-Analyse wurde auf einer 50 m CP-Sil 5/CB-Säule (Chrompack) durchgeführt. Temperaturprogramm: 150 °C, 3 min- 15 °C/min-300 °C, 45 min. Injektor: 280 °C. Detektor (FID): 300 °C

### DC-Bedingungen:

- DC-Platten: Kieselgel 60
- Laufmittel: 30 % Methyl-tert-butylether/n-Heptan
- Sprühreagenz: Cer(IV)sulfat-Molybdatophosphorsäure

### Beispiel 2: Bestimmung der Cholesterinsynthese in HepG2-Zellen (siehe Fahrner, J. et al., Eur. J. Biochem. 213: 1067-1073 (1993); Gebhardt, R., Lipids 28: 613-619 (1993); Pill, J. et al., Z. Anal. Chem. 332: 512-513 (1985)

Für den Nachweis einer Beeinflussung der Cholesterin-Biosynthese durch Testsubstanzen wurden HepG2-Zellkulturen eingesetzt. Diese Zellen wurden in 35 mm 6-well-Zellkulturinkubationsschalen in DMEM-Medium (Gibco, Eggenstein, Germany) mit 2 mM Gentamin, 10 % fötales Kälberserum, 40 U/ml Streptomycin und 50 U/ml Penicillin bis zur Konfluenz kultiviert. Eingefrorene Zellen wurden vor der Verwendung eine Woche kultiviert und passagiert. Die konfluenten Zellen wurden dann und in Serum-freiem Williams Medium E für 4 Stunden bei 37 °C mit den Testsubstanzen inkubiert. Dann wurde den Ansätzen ¹⁴C-Acetat (18,5 kBq/ml; 0,5 µCi/ml) zugefügt. Die Testsubstanzen wurden in DMSO verdünnt und in den angegebenen Konzentrationen eingesetzt. Nach weiteren 2 Stunden wurde das Medium entfernt, die Zellen zweimal mit physiologischer Salzlösung gewaschen, in destilliertes Wasser überführt und durch hochfrequenten Ultraschall homogenisiert (20 sec, Stufe 3).

Der Einbau von ¹⁴C-Acetat in die nicht verseifbare Lipid-Fraktion (Sterol-Fraktion) wurde durch folgende Methode bestimmt: 1,4 ml der Homogenate wurden mit 2,8 ml 0,5 M KOH in EtOH unter Schütteln für 1 Stunde bei 70 °C inkubiert. 2,1 ml dieser Menge wurden auf Extrelut®-Säulen (Merck, Darmstadt) aufgetragen. Die neutralen, lipophilen, nicht verseifbaren Substanzen wurden nach 30 min durch 10 ml n-Heptan in Szintillationsgefäße eluiert. Die ¹⁴C-Aktivität in den Proben wurde nach Zugabe von 10 ml Szintillationslösung (Ultima Gold@, Packard, Merident, Connecticut) in einem Szintillationszähler bestimmt. Die gezeigten Messwerte verstehen sich als Mittelwerte dreier unabhängiger Kulturschalen pro Versuchsansatz. Als Bezugswert wurde der Einbau von ¹⁴C-Acetat in Abwesenheit der Testsubstanzen bestimmt auf 100 % gesetzt.

### Versuchsergebnisse:

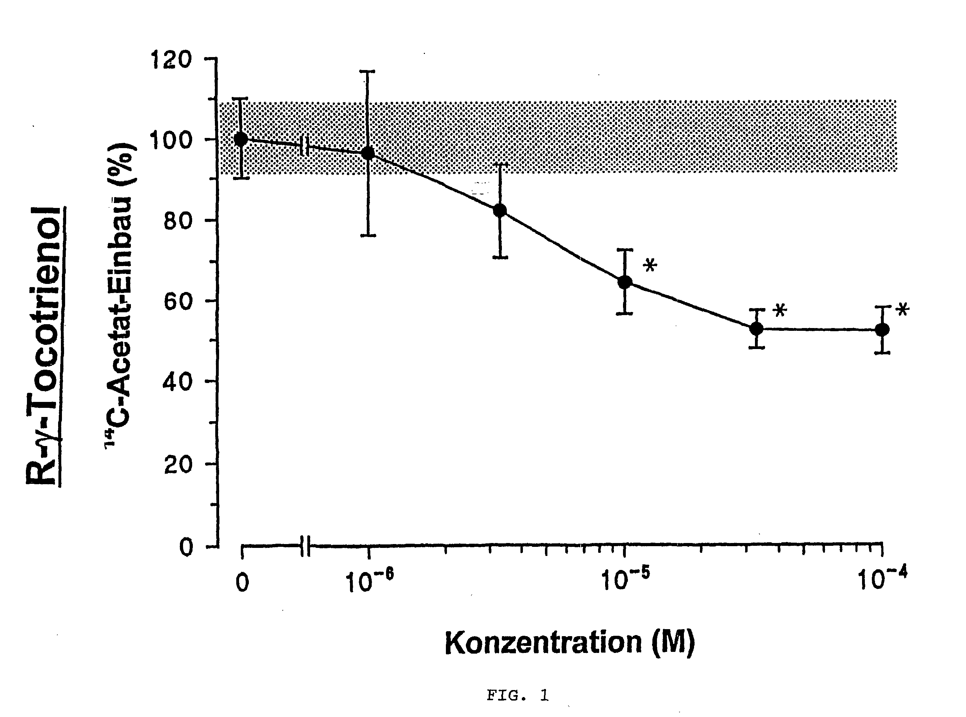

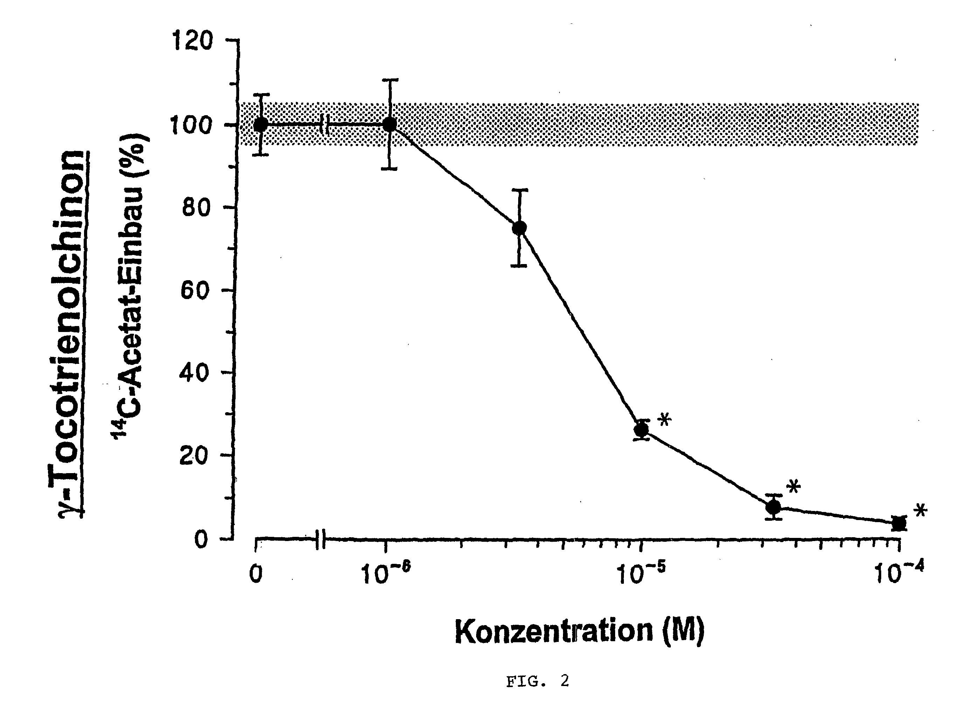

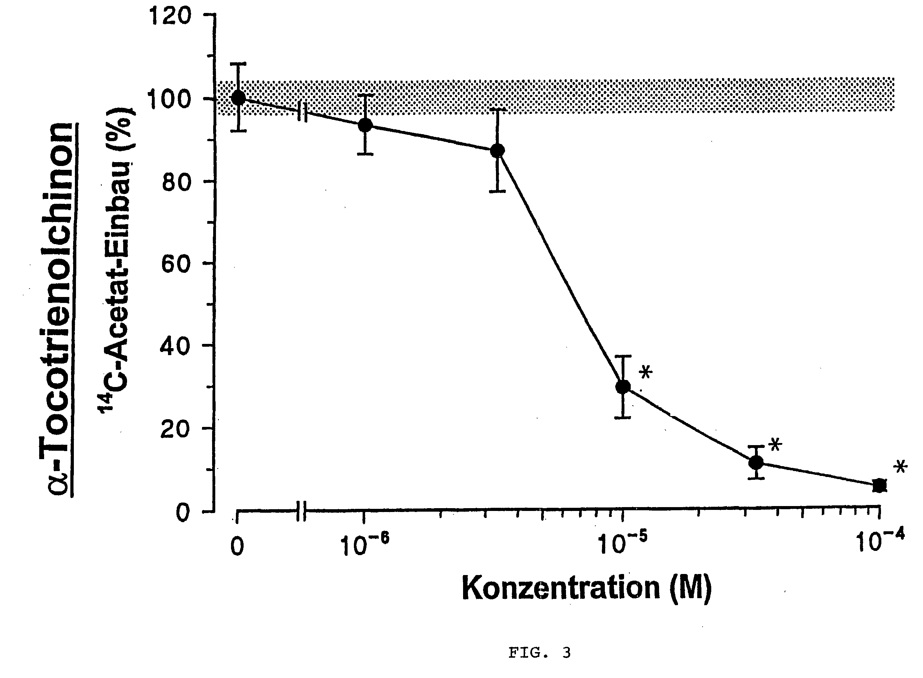

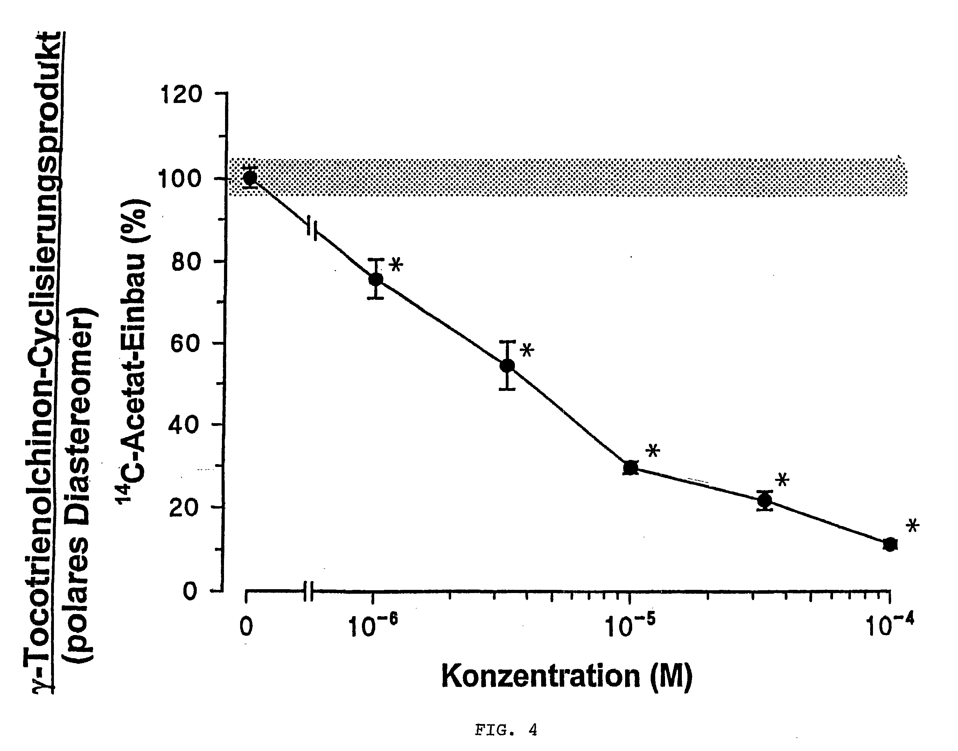

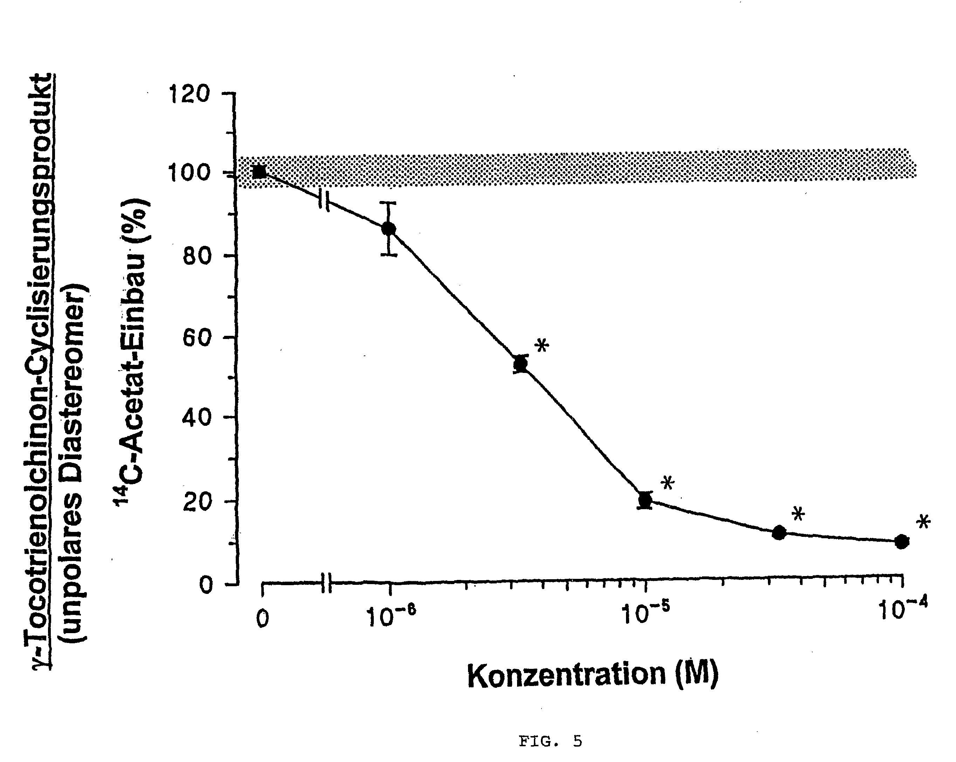

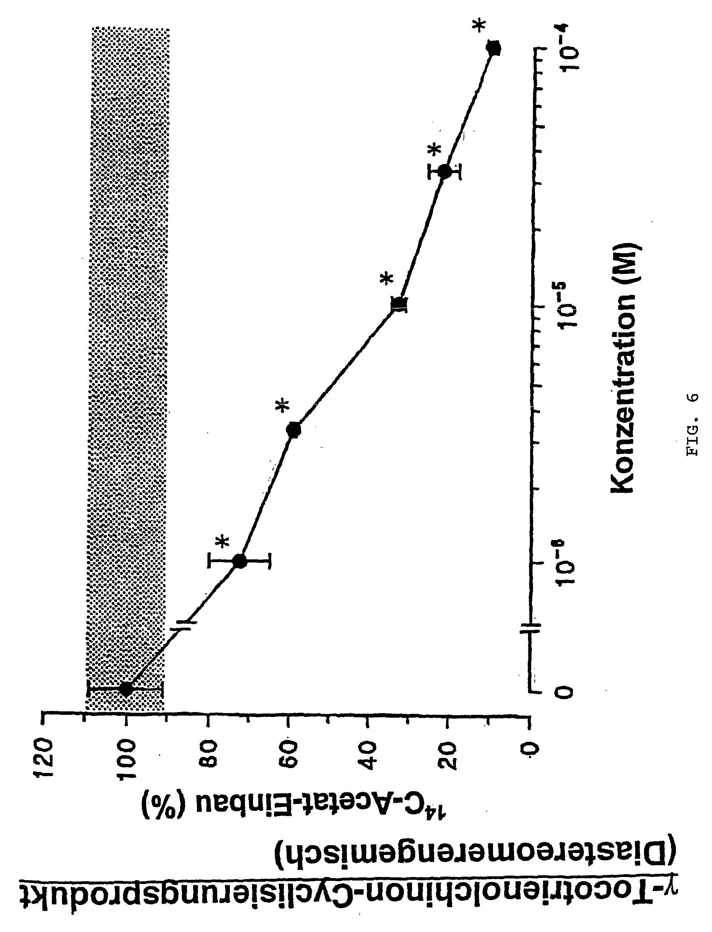

Folgende Substanzen wurden auf in Beispiel 2 angegebene Weise getestet: R-γ-Tocotrienol; γ-Tocotrienolchinon; α-Tocotrienolchinon; polares Diastereomer des γ-Tocotrienolchinon-Cyclisierungsprodukts, unpolares Diastereomer des γ-Tocotrienolchinon-Cyclisierungsprodukts, Racemat des γ-Tocotrienolchinon-Cyclisierungsprodukts. Die Ergebnisse sind in den Fig. 1 bis 6 dargestellt. Die in den Figuren angegebenen Werte entsprechen den Mittelwerten aus jeweils drei unabhängigen Messwerten. Sie sind ausgedrückt in relativen Einheiten bezogen auf den ¹⁴C-Acetat-Einbau in Zellen mit einer Zugabe der äquivalenten Menge DMSO als Kontrolle. R-γ-Tocotrienol bewirkt in einer Konzentration von 5 x 10⁻⁶ M eine ca. 20 %ige Hemmung des ¹⁴C-Acetat-Einbaus mit einer maximal ca. 50 %igen signifikanten Hemmung bei Konzentrationen ≥ 5 x 10⁻⁵ M (Fig. 1). γ- und α-Tocotrienolchinon bewirken erst ab einer Konzentration von 10⁻⁵ M eine signifikante, aber ca. 70- bis 75 %ige Hemmung des ¹⁴C-Acetat-Einbaus (Fig. 2 und 3). Sowohl das polare als auch das unpolare Diastereomer von γ-Tocotrienolchinon-Cyclisierungsprodukt sowie das Gemisch der beiden Diastereomere (1:1) bewirken bereits bei einer Konzentration von 10⁻⁶ M eine signifikante 20- bis 30 %ige Hemmung des ¹⁴C-Acetat-Einbaus, bei einer Konzentration von 5 x 10⁻⁵ eine ca. 50 %ige Hemmung (Fig. 4, 5 und 6). Damit sind die erfindungsgemäßen Cyclisierungsprodukte im Konzentrationsbereich von 10⁻⁶ M wesentlich wirksamer als γ-Tocotrienol und γ-Tocotrienolchinon.

## Patentansprüche

1. Verbindungen der Formel I 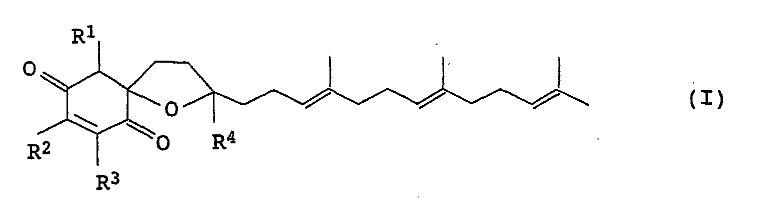 worin
R¹, R², R³ und R⁴ voneinander unabhängig für H oder C₁-C₆-Alkyl stehen; in stereoisomerenreiner Form oder als Gemisch von Stereoisomeren.

2. Verbindungen der Formel I, wobei R¹ für H oder CH₃, R² für H oder CH₃, R³ für CH₃ und R⁴ für H oder CH₃ steht.

3. Verfahren zur Herstellung einer Verbindung der Formel I, **dadurch gekennzeichnet, dass** man ein Tocotrienolchinon der Formel III 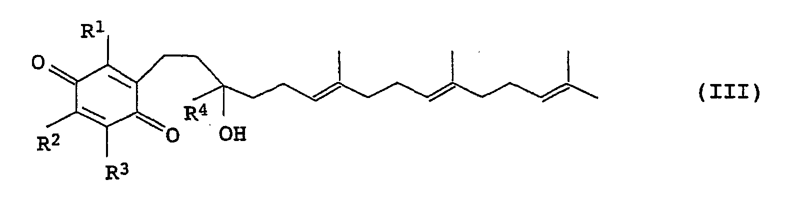 worin R¹ bis R⁴ die oben angegebenen Bedeutungen besitzen, in Gegenwart eines Katalysators zu einer Verbindung der Formel I umsetzt.

4. Verfahren nach Anspruch 3, **dadurch gekennzeichnet, dass** man Tocotrienolchinon der Formel III durch Oxidation eines Tocotrienols der Formel II 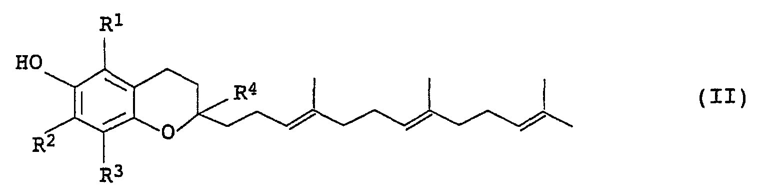 worin R¹ bis R⁴ die oben angegebenen Bedeutungen besitzen, herstellt.

5. Verfahren gemäß Anspruch 4, **dadurch gekennzeichnet, dass** als Oxidationsmittel Ce(IV)-Salze oder Fe(III)-Salze verwendet werden.

6. Verfahren gemäß Anspruch 4 oder 5, **dadurch gekennzeichnet, dass** die Oxidation des Tocotrienols der Formel II zum Tocotrienolchinon der Formel III in einem organischen Lösungsmittel durchgeführt wird.

7. Verfahren gemäß einem der Ansprüche 3 bis 6, **dadurch gekennzeichnet, dass** als Katalysator ein Alkalicarbonat verwendet wird.

8. Verwendung von Verbindungen der Formel I gemäß Anspruch 1 oder 2 als Nahrungsergänzungsmittel.

9. Verbindungen der Formel I gemäß Anspruch 1 oder 2 zur medizinischen Anwendung.

10. Verwendung einer Verbindung der Formel I gemäß Anspruch 1 oder 2 zur Herstellung eines Medikaments zur Behandlung und/oder Vorbeugung von Krankheiten im Zusammenhang mit Hypercholesterinämie wie kardiovaskuläre Erkrankungen einschließlich Arteriosklerose, Thrombose und Herzinfarkt oder zur Behandlung und/oder Vorbeugung von Krebserkrankungen.

11. Verwendung einer Verbindung der Formel I gemäß Anspruch 1 oder 2 zur Herstellung eines Medikaments zur Senkung des Plasmacholesterinspiegels beim Menschen.

12. Verwendung einer Verbindung der Formel I gemäß Anspruch 1 oder 2 zur Herstellung eines Medikaments zur Senkung des Plasmacholesterinspiegels bei Säugetieren oder Vögeln.

13. Pharmazeutisches Mittel, enthaltend wenigstens eine Verbindung der Formel I gemäß Anspruch 1 oder 2 und wenigstens einen pharmazeutisch verträglichen Träger.

## Claims

1. A compound of the formula I 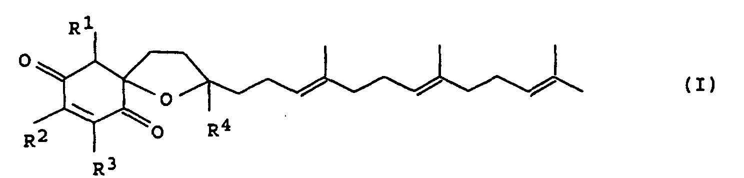 where
R¹, R², R³ and R⁴ independently of one another are H or C₁-C₆-alkyl; in stereoisomerically pure form or as a mixture of stereoisomers.

2. A compound of the formula I, where R¹ is H or CH₃, R² is H or CH₃, R³ is CH₃ and R⁴ is H or CH₃.

3. A process for preparing a compound of the formula I, which comprises reacting a tocotrienolquinone of the formula III 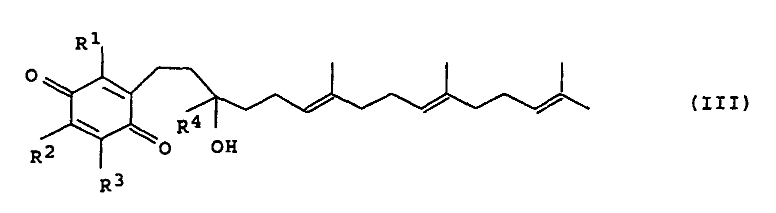 where R¹ to R⁴ have the meanings specified above, in the presence of a catalyst to give a compound of the formula I.

4. A process as claimed in claim 3, wherein tocotrienolquinone of the formula III is prepared by oxidizing a tocotrienol of the formula II 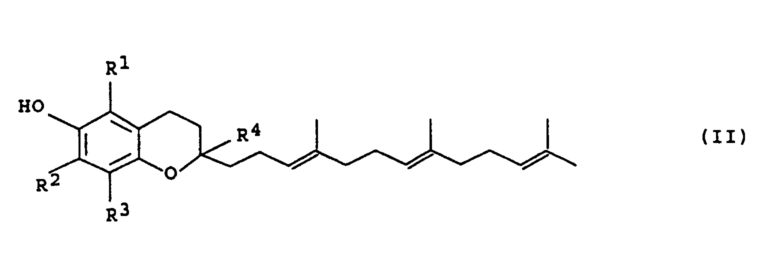 where R¹ to R⁴ have the meanings specified above.

5. A process as claimed in claim 4, wherein the oxidizing agent is Ce(IV) salts or Fe(III) salts.

6. A process as claimed in claim 4 or 5, wherein the tocotrienol of the formula II is oxidized to the tocotrienolquinone of the formula III in an organic solvent.

7. A process as claimed in one of claims 3 to 6, wherein the catalyst is an alkali metal carbonate.

8. The use of compounds of the formula I as claimed in claim 1 or 2 as food supplements.

9. A compound of the formula I as claimed in claim 1 or 2 for medical use.

10. The use of a compound of the formula I as claimed in claim 1 or 2 for preparing a medicament for treating and/or preventing disorders in relation to hypercholesterolemia such as cardiovascular disorders including arteriosclerosis, thrombosis and myocardial infarction or for treating and/or for preventing cancers.

11. The use of a compound of the formula I as claimed in claim 1 or 2 for preparing a medicament for lowering the plasma cholesterol level in humans.

12. The use of a compound of the formula I as claimed in claim 1 or 2 for preparing a medicament for lowering the plasma cholesterol level in mammals or birds.

13. A pharmaceutical composition comprising at least one compound of the formula I as claimed in claim 1 or 2 and at least one pharmaceutically compatible vehicle.

## Revendications

1. Composés de formule I 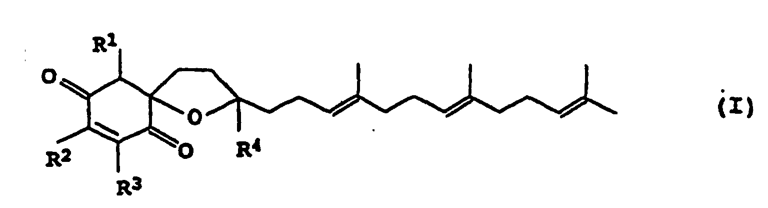 où
R¹, R², R³ et R⁴ désignent, indépendamment l'un de l'autre, H ou un alkyle en C₁-C₆; sous forme pure du point de vue des stéréoisomères ou comme mélange de stéréoisomères.

2. Composés de formule I, dans lesquels R¹ désigne H ou CH₃, R² représente H ou CH3, R³ représente CH₃ et R⁴ désigne H ou CH₃.

3. Procédé pour la préparation d'un composé de formule I, **caractérisé par le fait qu'**on convertit en un composé de formule I une tocotriénolquinone de formule III 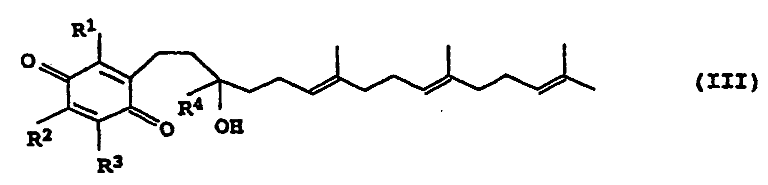 où R¹ à R⁴ possèdent les significations indiquées plus haut, en présence d'un catalyseur.

4. Procédé selon la revendication 3, **caractérisé par le fait qu'**on prépare la tocotriénolquinone de formule III par oxydation d'un tocotriénol de formule II 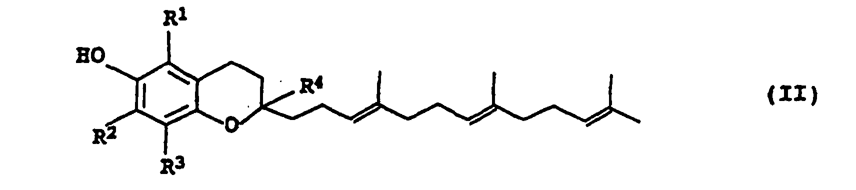 où R¹ à R⁴ possèdent les significations indiquées plus haut.

5. Procédé selon la revendication 4, **caractérisé par le fait qu'**on utilise comme agent d'oxydation des sels de Ce(IV) ou des sels de Fe(III).

6. Procédé selon la revendication 4 ou 5, **caractérisé par le fait que** l'oxydation du tocotriénol de formule II en tocotriénolquinone de formule III est réalisée dans un solvant organique.

7. Procédé selon l'une des revendications 3 à 6, **caractérisé par le fait qu'**on utilise comme catalyseur un carbonate alcalin.

8. Utilisation de composés de formule I selon la revendication 1 ou 2 comme complément alimentaire.

9. Composés de formule I selon la revendication 1 ou 2 pour utilisation médicale.

10. Utilisation d'un composé de formule I selon la revendication 1 ou 2 pour la préparation d'un médicament pour le traitement et/ou la prévention de maladies en rapport avec l'hypercholestérolémie, telles que les affections cardio-vasculaires, notamment l'artériosclérose, la thrombose ou l'infarctus du myocarde, ou pour le traitement et/ou la prévention des affections cancéreuses.

11. Utilisation d'un composé de formule I selon la revendication 1 ou 2 pour la préparation d'un médicament pour abaisser le niveau de cholestérol plasmatique chez l'homme.

12. Utilisation d'un composé de formule I selon la revendication 1 ou 2 pour la préparation d'un médicament pour abaisser le niveau de cholestérol plasmatique chez les mammifères ou les oiseaux.

13. Produit pharmaceutique, contenant au moins un composé de formule I selon la revendication 1 ou 2 et au moins un support pharmaceutiquement acceptable.
